# EUROPÄISCHE PATENTSCHRIFT

(11) **EP 0 816 327 B1**
(45) Veröffentlichungstag und Bekanntmachung des Hinweises auf die Patenterteilung: **17.01.2001**
(21) Anmeldenummer: 97109738.1
(22) Anmeldetag: 16.06.1997
(51) Int. Cl.: C07C 229/56, C07C 227/18

(54) **Verfahren zur Herstellung von N-(2-Carboxy-5-chlor-phenyl)glycin**
Process for preparing N-(2-carboxy-5-chloro-phenyl)glycine
Procédé pour la préparation de N-(carboxy-2 chloro-5 phényl)glycine

(30) Priorität: 20.06.1996 DE 19624583
(43) Veröffentlichungstag der Anmeldung: 07.01.1998
(73) Patentinhaber: Clariant GmbH, 65929 Frankfurt am Main (DE)
(72) Erfinder: Krause, Stefan, Dr., 65843 Sulzbach/Ts (DE); Neumann-Grimm, Doris, Dr., 60388 Frankfurt (DE); Papenfuhs, Theodor, Dr., 60433 Frankfurt (DE)

(56) Entgegenhaltungen:
- WO-A-97/08129
- DE-C- 142 507
- BEILSTEINS HANDBUCH DER ORGANISCHEN CHEMIE, 4. Auflage, 2. Ergänzungswerk Band 14, 1951, SPRINGER-VERLAG, Berlin * Seite 231, Zeilen 6-16 *
- BEILSTEINS HANDBUCH DER ORGANISCHEN CHEMIE, 4. Auflage, Band 14, 1931 VERLAG VON JULIUS SPRINGER, Berlin * Seite 366, Zeilen 17-22 *
- BEILSTEINS HANDBUCH DER ORGANISCHEN CHEMIE, 4. Auflage, 4. Ergänzungswerk Band 14, 1985, SPRINGER-VERLAG, Berlin * Seite 1074, Zeilen 14-19 *

## Beschreibung

Die vorliegende Erfindung betrifft ein gegenüber dem Stand der Technik verbessertes Verfahren zur Herstellung von N-(2-Carboxy-5-chlor-phenyl)glycin.

2-Carboxyphenylglycine besitzen unter anderem eine Bedeutung für die technische Herstellung von Indigo. Sie lassen sich gemäß Ullmann's Encyklopedia of Industrial Chemistry, Vol. A. 14, S. 105 bis 151 durch Umsetzung von Anthranilsäure mit Chloressigsäure herstellen. Diese Art der Synthese ist jedoch problematisch, da die Aminogruppe in der Anthranilsäure mit der Chloressigsäure nicht nur einfach, sondern auch zweifach reagieren kann und somit zur Bildung zweifach alkylierter Produkte führt.

Ausgehend von N-(2-Carboxy-5-chlor-phenyl)glycin ist 6,6'-Dichlorindigo zugänglich. Es ist bekannt, daß die Substituenten am Indigogerüst Einfluß auf die Absorptionsmaxima des Indigos nehmen. So weist, wie aus H. Zollinger, Color chemistry: syntheses, properties and applications of organic dyes and pigments, VCH Verlagsgesellschaft Weinheim - New York 1987, Seiten 152 bis 159 hervorgeht, das 6,6'-Dichlorindigo ein Absorptionsmaximum für die längste Wellenlänge bei 590 nm auf (siehe auch Table 8-1 auf Seite 154).

Eine weitere ebenfalls in Ullmann's Encyklopedia beschriebene Synthese besteht darin, Anthranilsäure mit Formaldehyd und Cyanwasserstoff umzusetzen. Gegen diese Herstellung spricht allerdings die Verwendung des sehr giftigen Cyanwasserstoffs (Blausäure).

Die DE-PS 142 507 betrifft die Herstellung von Phenylglycin-o-carbonsäure (2-Carboxyphenylglycin) durch Umsetzung des Kaliumsalzes von 2-Chlorbenzoesäure mit Glycin in Gegenwart von Kaliumhydroxid, Kaliumcarbonat und katalytischen Mengen an Kupfer. Hierbei werden zunächst alle Reaktionskomponenten in Wasser gelöst vorgelegt und anschließend erhitzt. Bei Durchführung dieser Reaktion ergeben sich unter Verwendung von 2,4-Dichlorbenzoesäure jedoch Schwierigkeiten. Zum einen bilden sich in größeren Mengen Nebenprodukte, zum anderen führt die Umsetzung nicht zu reproduzierbaren Ergebnissen, da die Umsetzung häufig nicht vollständig abläuft, sondern ohne erkennbaren Grund nur zu einem vergleichsweise geringen Teilumsatz führt und anschließend stehen bleibt.

In Hinblick auf die vorstehend geschilderten Nachteile, besteht ein Bedarf an einem Verfahren, das die vorstehend geschilderten Nachteile vermeidet und sich zudem einfach und ohne einen großen technischen Aufwand realisieren läßt. Das Verfahren soll das gewünschte Produkt in hohen Ausbeuten liefern und sich zudem sicher reproduzieren lassen.

Gelöst wird diese Aufgabe durch ein Verfahren zur Herstellung von M-(2-Carboxy-5-chlor-phenyl)glycin der Formel (1)

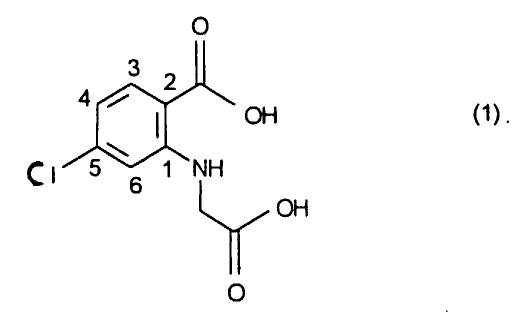

Es ist dadurch gekennzeichnet, daß man 2,4-Dichlorbenzoesäure der Formel (2) 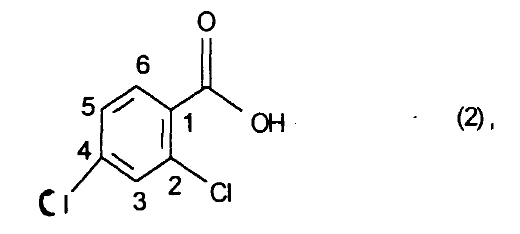 mit Glycin und einer Base in Wasser bei einer Temperatur von 50 bis 200°C in Gegenwart von Kupfer und Sauerstoff bei einem pH-Wert von 7 bis 13 umsetzt.

Das erfindungsgemäße Verfahren besitzt mehrere Vorteile. Es geht zum einen von vergleichsweise leicht zugänglichen Ausgangsstoffen aus und läßt sich auf einfache Art und Weise durchführen. Darüber hinaus liefert es, bezogen auf die eingesetzten Ausgangsstoffe, das gewünschte N-(2-Carboxy-5-chlorphenyl)glycin in hohen bis sehr hohen Ausbeuten. Dabei ist gewährleistet, daß das Verfahren hinsichtlich Umsatz, Selektivität und somit Ausbeute stets zu reproduzierbaren Ergebnissen führt und es nicht dem Zufall überlassen bleibt, ob und in welchem Umfange die Umsetzung zum gewünschten Produkt abläuft.

Bei Durchführung chemischer Prozesse arbeitet man in der Regel unter Ausschluß von Sauerstoff respektive Luft. Dies trifft insbesondere bei der Realisierung chemischer Synthesen im technischen Maßstäbe zu. Wendet man eine derartige Arbeitsweise auf die Herstellung von N-(2-Carboxy-5-chlorphenyl)glycin an und führt die Umsetzung der 2,4-Dichlorbenzoesäure der Formel (2) mit Glycin und einer Base in Wasser unter Ausschluß von Sauerstoff respektive Luft durch, so schreitet die Reaktion nur bis zu einem bestimmten Umsetzungsgrad fort und gelangt dann zum Stillstand. Obwohl noch nicht umgesetzte Ausgangsstoffe vorhanden sind und obwohl die Reaktionsbedingungen unverändert beibehalten werden, läßt sich - wie ein entsprechender Vergleichsversuch beweist - die Reaktion nicht weiter fortsetzen und die Ausbeute des erwünschten N-(2-Carboxy-5-chlor-phenyl)glycins nicht mehr steigern.

Das gleiche Verhalten kann man beobachten, wenn man die Reaktanten in das Reaktionsgefäß, beispielsweise in einem Glaskolben, einfüllt und die Umsetzung in Anwesenheit der im Reaktionsgefäß noch vorhandenen Luft durchführt. Auch hierbei verläuft die Umsetzung - wie Vergleichsversuche zeigen - in nicht reproduzierbarer Weise bis zu einem gewissen Grade und bleibt dann ebenfalls stehen.

Die Umsetzung läßt sich jedoch in den beiden vorstehend beschriebenen Fällen unerwarteterweise weiterführen, wenn man der Reaktion Sauerstoff, respektive Luft, zuleitet und die Umsetzung somit in Anwesenheit von Sauerstoff, respektive Luft, fortsetzt.
Dieses Verhalten deutet darauf hin, daß erst die Anwesenheit einer ausreichenden Menge Sauerstoff, respektive Luft, während der gesamten Reaktion sicherstellt, daß die Umsetzung im gewünschten Umfange und in reproduzierbarer Weise abläuft. Dies war in keiner Weise vorherzusehen, und es ist als sehr überraschend zu betrachten, daß die Gegenwart von Sauerstoff die Umsetzung offensichtlich günstig beeinflußt.

Setzt man 2,4-Dichlorbenzoesäure mit Glycin analog zur DE-PS 142 507 um, indem man alle Reaktanten vorlegt und die Reaktion anschließend unter Rühren und Erhitzen durchführt, so bilden sich - wie ein entsprechender Vergleichsversuch belegt - in nicht unerheblichem Umfange Nebenprodukte, insbesondere 4-Chlorsalicylsäure. Die in der DE-PS 142 507 getroffene Aussage, die Ausbeute sei bei Verwendung von 2-Chlorbenzoesäure fast theoretisch, kann bei Verwendung von 2,4-Dichlorbenzoesäure als Ausgangsstoff nicht bestätigt werden.

Die Bildung von Nebenprodukten, insbesondere 4-Chlorsalicylsäure, läßt sich jedoch überraschenderweise deutlich vermindern, indem man die Umsetzung, wie zuvor angegeben, bei einem bestimmten pH-Wert durchführt. Das läßt sich besonders einfach realisieren, wenn man die Base der Reaktion in dem Maße zuführt, daß der gewünschte pH-Wert eingehalten wird.

Zur Durchführung des erfindungsgemäßen Verfahrens ist es erforderlich die Umsetzung der 2,4-Dichlorbenzoesäure der Formel (2) mit Glycin in Wasser sowohl in Anwesenheit von Sauerstoff als auch bei dem zuvor genannten pH-Wert durchzuführen. Diese beiden Maßnahmen sind einzuhalten, um den gewünschten Erfolg zu gewährleisten.

Man ist hinsichtlich der Zugabe der Reaktionspartner an keine besondere Reihenfolge gebunden, es ist lediglich sicherzustellen, daß während der Umsetzung alle an der Reaktion beteiligten Reaktanten anwesend sind.

Lediglich bei Verwendung von Carbonaten in Mischung mit Hydroxiden sollten die Carbonate als letzte zugegeben werden.

Üblicherweise legt man die 2,4-Dichlorbenzoesäure der Formel (2), Glycin und Wasser vor, und setzt anschließend einen Teil der Base zu, um aus der 2,4-Dichlorbenzoesäure und dem Glycin die entsprechenden Salze zu bilden. Das für die Umsetzung als Katalysator benötigte Kupfer kann man zuvor oder auch nachher zusetzen.

Das Wasser dient sowohl für die 2,4-Dichlorbenzoesäure, Glycin und deren Salze, als auch für das in Form von Salzen anfallende N-(2-Carboxy-5-chlorphenyl)glycin als Lösungsmittel. Man setzt Wasser in dem Maße ein, daß sich die vorstehend genannten Stoffe darin vollständig oder zum Teil lösen und zudem eine Lösung oder eine Suspension mit nicht zu hoher Viskosität gebildet wird, um eine gute Rührbarkeit des Reaktionsgemisches zu gewährleisten.

Üblicherweise verwendet man Wasser zu 2,4-Dichlorbenzoesäure im Gewichtsverhältnis (0,5 bis 10):1, insbesondere (0,7 bis 5):1, bevorzugt (1 bis 3):1. Man kann auch mit größeren Wassermengen, beispielsweise bis 15:1 oder 20:1, arbeiten, erhält hierbei jedoch verhältnismäßig große Volumina wäßriger Lösungen, die gehandhabt werden müssen.

Es ist allerdings auch möglich, direkt die entsprechenden Salze der 2,4-Dichlorbenzoesäure und des Glycins zu verwenden und diese in Wasser zu lösen. Auch in diesem Falle ist es erforderlich, eine ausreichende Menge Wasser zur Verfügung zu stellen, damit sich die Salze der 2,4-Dichlorbenzoesäure und die des Glycins ganz oder zum Teil lösen und eine Lösung, beziehungsweise Suspension mit nicht zu großer Viskosität gebildet wird.

Die für die Umsetzung benötigen Reaktanten lassen sich in einem breiten Molverhältnis umsetzen. Üblicherweise setzt man die 2,4-Dichlorbenzoesäure der Formel (2) und Glycin im Molverhältnis 1:1 bis 1:2, insbesondere 1:1 bis 1:1,5 um.

Als Base lassen sich alle Stoffe, die eine hinreichende Basizität aufweisen, verwenden. Mit gutem Erfolg kann man als Base ein Oxid, Hydroxid, Carbonat oder Hydrogencarbonat eines Alkalimetalls oder Erdalkalimetalls oder ein Gemisch derselben, insbesondere ein Oxid, Hydroxid oder Carbonat eines Alkalimetalls oder ein Gemisch derselben, bevorzugt Natriumhydroxid, Kaliumhydroxid, Natriumcarbonat oder Kaliumcarbonat oder ein Gemisch derselben, einsetzen. Besonders bevorzugt ist ein aus Natriumhydroxid/Natriumcarbonat und/oder Kaliumhydroxid/Kaliumcarbonat bestehendes Gemisch.

Wie bereits erwähnt, läßt sich die Umsetzung in einem Temperaturbereich von 50 bis 200°C durchführen.

In einer Vielzahl von Fällen genügt es, die Umsetzung bei einer Temperatur von 60 bis 160, insbesondere 90 bis 130°C durchzuführen.

Zur Durchführung der Umsetzung wird Kupfer als Katalysator benötigt.

Als Kupfer kann man metallisches Kupfer, beispielsweise in Form von Spänen, Pulver oder Kupferbronze, eine Kupfer(I)- oder Kupfer(II)-Verbindung oder ein Gemisch derselben, insbesondere ein Kupfer(I)-Salz oder ein Kupfer(II)-Salz oder ein Gemisch derselben, einsetzen.

Ohne Anspruch auf Vollständigkeit zu erheben seien als Kupfer(I)- und Kupfer(II)-Verbindungen, Kupfer(I)chlorid, Kupfer(II)chlorid, basisches Kupfercarbonat, Kupfer(I)oxid, Kupfer(II)oxid, Kupfercarbonat, Kupfer(II)nitrat, Kupfer(II)sulfat, Kupferacetat, Kupferacetylacetonat genannt.

Zur Durchführung des Verfahrens genügt es in den meisten Fällen, der Reaktion 0,005 bis 2,5, insbesondere 0,02 bis 1,5, bevorzugt 0,05 bis 0,5 Gewichts% Kupfer (gerechnet als elementares oder chemisch gebundenes Kupfer), bezogen auf 2,4-Dichlorbenzoesäure zuzusetzen. Es ist allerdings auch möglich die Umsetzung in Anwesenheit größerer Kupfermengen, beispielsweise bis 4 oder 5 Gewichts%, bezogen auf 2,4-Dichlorbenzoesäure der Formel (2) ablaufen zu lassen. In der Regel führen jedoch derartig hohe Kupfermengen zu keinem besonderen Vorteil.

Wie eingangs erläutert, erfordert das erfindungsgemäße Verfahren die Anwesenheit von Sauerstoff. Man setzt als Sauerstoff reinen Sauerstoff oder ein Sauerstoff enthaltendes Gas ein. Hierbei ist lediglich zu beachten, daß der Sauerstoff in ausreichendem Maße zur Verfügung gestellt wird. Dies läßt sich besonders einfach sicherstellen, indem man während der Umsetzung reinen Sauerstoff oder ein Sauerstoff enthaltendes Gas durch das Reaktionsgemisch leitet. Üblicherweise setzt man als Sauerstoff enthaltendes Gas Luft ein.

Das erfindungsgemäße Verfahren läßt sich üblicherweise in einem vergleichweise großen pH-Bereich, nämlich bei einem pH-Wert von 7 bis 13 durchführen. Hierbei ist jedoch auch zu berücksichtigen, daß der jeweils zu wählende pH-Wert in gewissem Umfange auch von der jeweils gewählten Reaktionstemperatur und von der Konzentration der 2,4-Dichlorbenzoesäure der Formel (2) abhängig sein kann. So kann es durchaus sinnvoll sein, in einer Vielzahl von Fällen bei vergleichsweise niedrigen pH-Werten zu arbeiten, um die Bildung von Nebenprodukten zurückzudrängen.

Bei einer Vielzahl von Fällen hat es sich bewährt, die Umsetzung bei einem pH-Wert von 7,5 bis 11, insbesondere 8 bis 10 durchzuführen.

Der vorgegebene pH-Wert läßt sich besonders einfach einhalten, in dem man den pH-Wert durch Zugabe der Base einstellt. Da im Verlauf der Umsetzung die eingesetzte Base infolge der Neutralisation von freigesetzter Salzsäure verbraucht wird, setzt man die Base üblicherweise der Reaktion in dem Maße zu, daß die Einhaltung des pH-Wertes gewährleistet wird.

Zum Einstellen eines konstanten pH-Wertes kann man die Lauge so dosieren, daß der pH-Wert gemessen und bei Abweichung vom Soll-pH-Wert nach unten Lauge zugegeben wird.
Wenn durch Vorversuche die Reaktionsgeschwindigkeit beziehungsweise der Laugenverbrauch in Abhängigkeit von der Zeit ermittelt wurde, kann aufgrund dieser Ergebnisse auch ein festes, vorgegebenes Programm für die Laugendosierung verwendet werden, um einen pH-Wert im gewünschten Bereich einzustellen.

Nach Beendigung der Umsetzung liegt das N-(2-Carboxy-5-chlor-phenyl)glycin der Formel (1) üblicherweise in Form einer wäßrigen Lösung oder Suspension seiner Salze vor. Um das freie N-(2-Carboxy-5-chlor-phenyl)glycin zu erhalten, säuert man die wäßrige Lösung durch Zugabe einer Säure, beispielsweise einer Mineralsäure, an. Aus der angesäuerten wäßrigen Lösung fällt das freie N-(2-Carboxy-5-chlor-phenyl)glycin aus. Durch Zugabe einer ausreichenden Säuremenge ist sicherzustellen, daß die Salze des N-(2-Carboxy-5-chlorphenyl)glycins vollständig in das freie N-(2-Carboxy-5-chlor-phenyl)glycin überführt werden.

In einer Vielzahl von Fällen erweist es sich als ausreichend, durch Zugabe der Säure einen pH-Bereich von 0 bis 4, insbesondere 0 bis 2 einzustellen. Eine Zugabe von Säure bis zu einem pH-Wert von -0,5 ist möglich, aber wegen der erforderlichen großen Mengen an Säure nicht in jedem Falle erwünscht.

Um eine gut rührbare Suspension zu erhalten, gibt man vorteilhafterweise das Reaktionsgemisch zu der notwendigen Menge Säure.

Das ausgefallene N-(2-Carboxy-5-chlor-phenyl)glycin läßt sich durch Filtration oder Extraktion, insbesondere durch Filtration abtrennen. Durch Trocknen des abfiltrierten N-(2-Carboxy-5-chlor-phenyl)glycins erhält man üblicherweise Produkte in ausreichender Reinheit.

Falls eine noch höhere Reinheit erwünscht sein sollte, so läßt sich das N-(2-Carboxy-5-chlor-phenyl)glycin durch Umkristallisieren oder nochmaliges Ausfällen aus einer wäßrigen Lösung seiner Salze zusätzlich reinigen.

Das Verfahren läßt sich unter reduziertem Druck, bei Normaldruck und unter Überdruck durchführen. Es eignet sich insbesondere für eine Durchführung bei Normaldruck.

Das Verfahren läßt sich kontinuierlich oder diskontinuierlich durchführen.

Besonders einfach gestaltet sich eine diskontinuierliche Durchführung des Verfahrens.

Die nachfolgenden Beispiele belegen die Erfindung, ohne sie zu beschränken.

### Experimenteller Teil

### Beispiel 1

### Herstellung von N-(2-Carboxy-5-chlor-phenyl)glycin in Gegenwart von Sauerstoff und unter Kontrolle des pH-Wertes

Man trägt in einen mit Rückflußkühler, pH-Elektrode und einer Pumpvorrichtung mit pH-Steuerung bestückten Dreihalskolben (Volumen: 1 Liter) 250 g Wasser, 1 mol KOH in Form von 65 g einer 86 %igen wäßrigen Lösung, 191 g (1 mol) 2,4-Dichlorbenzoesäure, 83 g (1,1 mol) Glycin, 76 g (0,55 mol) Kaliumcarbonat und 200 mg Kupferpulver unter Rühren ein. Die Apparatur wird evakuiert und nachfolgend mit Sauerstoff belüftet. Anschließend erhitzt man die wäßrige Lösung bis zum Rückfluß (106°C) und pumpt 50 %ige wäßrige Kaliumhydroxidlösung so zu, daß der pH-Wert in der wäßrigen Lösung bei 9 gehalten wird. Nach etwa drei Stunden wird kein Kaliumhydroxid mehr verbraucht, wodurch das Ende der Umsetzung angezeigt wird.

Das Reaktionsgemisch weist nach Ansäuern auf pH 1 und nach Veresterung mit Diazomethan gemäß gaschromatographischer Analyse folgende Zusammensetzung auf:

| | |
|---|---|
| 2,4-Dichlorbenzoesäure* | 1,5 Gew.-% |
| 4-Chlorsalicylsäure* | 6,0 Gew.-% |
| 4-Chlorbenzoesäure* | 1,5 Gew.-% |
| N-(2-Carboxy-5-chlor-phenyl)glycin* | 91,0 Gew.-% |

| | |
|---|---|
| * als Methylester respektive Methoxyverbindung | |

### Beispiel 2

### Herstellung von N-(2-Carboxy-5-chlor-phenyl)glycin in Gegenwart von Sauerstoff und unter Kontrolle des pH-Wertes

Man arbeitet wie in Beispiel 1 beschrieben, setzt anstelle von 200 mg Kupferpulvers jedoch 350 mg basisches Kupfercarbonat ein.

Das Reaktionsgemisch weist nach Ansäuren auf pH 1 und nach Veresterung mit Diazomethan gemäß gaschromatographischer Analyse folgende Zusammensetzung auf:

| | |
|---|---|
| 2,4-Dichlorbenzoesäure* | 1 Gew.-% |
| 4-Chlorsalicylsäure* | 6 Gew.-% |
| 4-Chlorbenzoesäure* | 2 Gew.-% |
| N-(2-Carboxy-5-chlor-phenyl)glycin | 91 Gew.-% |

| | |
|---|---|
| * als Methylester respektive Methoxyverbindung | |

### Vergleichsbeispiel 1

### Herstellung von N-(2-Carboxy-5-chlor-phenyl)glycin in Abwesenheit von Sauerstoff aber unter Kontrolle des pH-Wertes

Man trägt, wie in Beispiel 1 beschrieben, in einen mit Rückflußkühler, pH-Elektrode und einer Pumpvorrichtung mit pH-Steuerung bestückter Dreihalskolben (Volumen: 1 Liter) 250 g Wasser, 1 mol KOH in Form von 65 g einer 86 %iger wäßrigen Lösung, 191 g (1 mol) 2,4-Dichlorbenzoesäure, 83 g (1,1 mol) Glycin, 76 g (0,55 mol) Kaliumcarbonat und 200 mg Kupferpulver unter Rühren bei Raumtemperatur ein.

Die Apparatur wird mittels Ultraschall entgast, evakuiert und nachfolgend nicht mit Sauerstoff, wie in Beispiel 1 angegeben, sondern mit Argon belüftet.

Anschließend erhitzt man die von Sauerstoff befreite wäßrige Lösung bis zum Rückfluß (106°C) und pumpt 50 %ige wäßrige Kaliumhydroxidlösung so zu, daß der pH-Wert der wäßrigen Lösung bei 9 gehalten wird.

Nach drei Stunden ist im Vergleich zu Beispiel 1 wenig Kaliumhydroxid verbraucht worden und es wird kein Kaliumhydroxid mehr verbraucht, wodurch das Ende der Umsetzung angezeigt wird.

Das Reaktionsgemisch weist nach Ansäuern auf pH 1 und nach Veresterung mit Diazomethan gemäß gaschromatographischer Analyse folgende Zusammensetzung auf:

| | |
|---|---|
| 2,4-Dichlorbenzoesäure* | 87 % |
| 4-Chlorsalicylsäure* | 1 % |
| 4-Chlorbenzoesäure* | < 0,2 % |
| N-(2-Carboxy-5-chlor-phenyl)glycin* | 12 % |

| | |
|---|---|
| * als Methylester respektive Methoxyverbindung | |

### Vergleichsbeispiel 2

### Herstellung von N-(2-Carboxy-5-chlor-phenyl)glycin in Gegenwart von Sauerstoff, jedoch ohne Kontrolle des pH-Wertes

Man trägt in einen mit Rückflußkühler, pH-Elektrode und einer Pumpvorrichtung mit pH-Steuerung bestückten Dreihalskolben (Volumen: 1 Liter) 250 g Wasser, 2 mol KOH in Form von 124 g einer 90 %igen wäßrigen Lösung, 191 g (1 mol) 2,4-Dichlorbenzoesäure, 75 g (1 mol) Glycin, 76 g (0,55 mol) Kaliumcarbonat und 200 mg Kupferpulver unter Rühren bei Raumtemperatur ein. Die Apparatur wird evakuiert und nachfolgend mit Sauerstoff belüftet.

Anschließend erhitzt man die wäßrige Lösung 6 Stunden bis zum Ende der Umsetzung unter Rückfluß.

Das Reaktionsgemisch weist nach Ansäuern auf pH 1 und nach Veresterung mit Diazomethan gemäß gaschromatographischer Analyse folgende Zusammensetzung auf:

| | |
|---|---|
| 2,4-Dichlorbenzoesäure^{*} | 2 % |
| 4-Chlorsalicylsäure^{*} | 13 % |
| 4-Chlorbenzoesäure^{*} | 2 % |
| N-(2-Carboxy-5-chlor-phenyl)glycin^{*} | 83 % |

| | |
|---|---|
| * als Methylester respektive Methoxyverbindung | |

## Patentansprüche

1. Verfahren zur Herstellung von N-(2-Carboxy-5-chlor-phenyl)glycin der Formel (1) 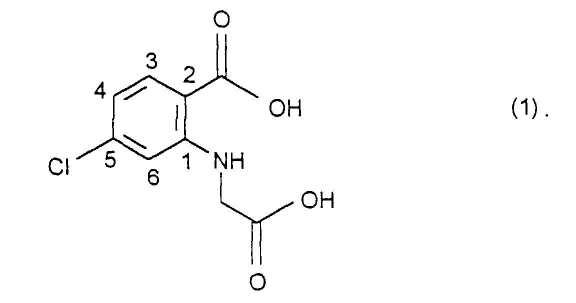 dadurch gekennzeichnet, daß man 2,4-Dichlorbenzoesäure der Formel (2) 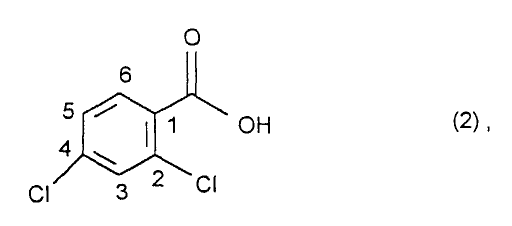 mit Glycin und einer Base in Wasser bei einer Temperatur von 50 bis 200°C in Gegenwart von Kupfer und Sauerstoff bei einem pH-Wert von 7 bis 13 umsetzt.

2. Verfahren nach Anspruch 1, dadurch gekennzeichnet, daß man 2,4-Dichlorbenzoesäure der Formel (2) und Glycin im Molverhältnis 1:1 bis 1:2 umsetzt.

3. Verfahren nach Anspruch 1 oder 2, dadurch gekennzeichnet, daß man als Base ein Oxid, Hydroxid, Carbonat oder Hydrogencarbonat eines Alkalimetalls oder Erdalkalimetalls oder ein Gemisch derselben einsetzt.

4. Verfahren nach einem oder mehreren der Ansprüche 1 bis 3, dadurch gekennzeichnet, daß man als Base ein Oxid, Hydroxid oder Carbonat eines Alkalimetalls oder ein Gemisch derselben einsetzt.

5. Verfahren nach einem oder mehreren der Ansprüche 1 bis 4, dadurch gekennzeichnet, daß man als Base Natriumhydroxid, Kaliumhydroxid, Natriumcarbonat oder Kaliumcarbonat oder ein Gemisch derselben einsetzt.

6. Verfahren nach einem oder mehreren der Ansprüche 1 bis 5, dadurch gekennzeichnet, daß man die Umsetzung bei einer Temperatur von 60 bis 160°C durchführt.

7. Verfahren nach einem oder mehreren der Ansprüche 1 bis 6, dadurch gekennzeichnet, daß man die Umsetzung bei einer Temperatur von 90 bis 130°C durchführt.

8. Verfahren nach einem oder mehreren der Ansprüche 1 bis 7, dadurch gekennzeichnet, daß man als Kupfer metallisches Kupfer, eine Kupfer(I)- oder Kupfer(II)-Verbindung oder ein Gemisch derselben einsetzt.

9. Verfahren nach einem oder mehreren der Ansprüche 1 bis 8, dadurch gekennzeichnet, daß man als Kupfer ein Kupfer(I)salz oder ein Kupfer(II)salz oder ein Gemisch derselben einsetzt.

10. Verfahren nach einem oder mehreren der Ansprüche 1 bis 9, dadurch gekennzeichnet, daß man 0,005 bis 2,5 Gewichts% Kupfer, bezogen auf 2,4-Dichlorbenzoesäure, einsetzt.

11. Verfahren nach einem oder mehreren der Ansprüche 1 bis 10, dadurch gekennzeichnet, daß man 0,05 bis 0,5 Gew.-% Kupfer, bezogen auf 2,4-Dichlorbenzoesäure, einsetzt.

12. Verfahren nach einem oder mehreren der Ansprüche 1 bis 11, dadurch gekennzeichnet, daß man als Sauerstoff reinen Sauerstoff oder ein Sauerstoff enthaltendes Gas einsetzt.

13. Verfahren nach einem oder mehreren der Ansprüche 1 bis 12, dadurch gekennzeichnet, daß man während der Umsetzung reinen Sauerstoff oder ein Sauerstoff enthaltendes Gas durch das Reaktionsgemisch leitet.

14. Verfahren nach einem oder mehreren der Ansprüche 1 bis 13, dadurch gekennzeichnet, daß man als Sauerstoff enthaltendes Gas Luft einsetzt.

15. Verfahren nach einem oder mehreren der Ansprüche 1 bis 14, dadurch gekennzeichnet, daß man die Umsetzung bei einem pH-Wert von 7,5 bis 11 durchführt.

16. Verfahren nach einem oder mehreren der Ansprüche 1 bis 15, dadurch gekennzeichnet, daß man die Umsetzung bei einem pH-Wert von 8 bis 10 durchführt.

17. Verfahren nach einem oder mehreren der Ansprüche 1 bis 16, dadurch gekennzeichnet, daß man den pH-Wert durch Zugabe der Base einstellt.

## Claims

1. A process for the preparation of N-(2-carboxy-5-chloro-phenyl)glycine of the formula (1) 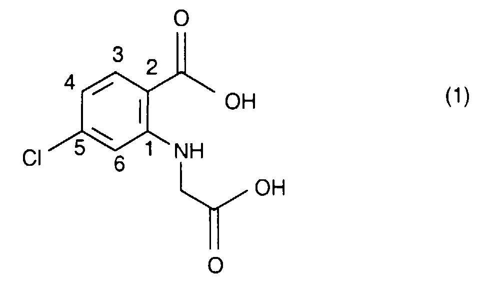 which comprises reacting 2,4-dichlorobenzoic acid of the formula (2) 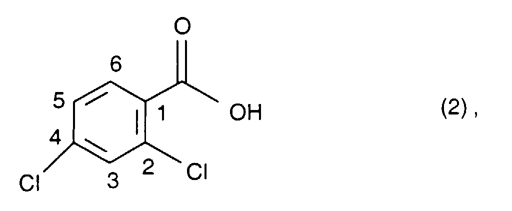 with glycine and a base in water at a temperature of 50 to 200°C in the presence of copper and oxygen at a pH of 7 to 13.

2. The process as claimed in claim 1, wherein 2,4-dichlorobenzoic acid of the formula (2) and glycine are reacted in a molar ratio of 1:1 to 1:2.

3. The process as claimed in claim 1 or 2, wherein an oxide, hydroxide, carbonate or bicarbonate of an alkali metal or alkaline earth metal or a mixture thereof is employed as the base.

4. The process as claimed in one or more of claims 1 to 3, wherein an oxide, hydroxide or carbonate of an alkali metal or a mixture thereof is employed as the base.

5. The process as claimed in one or more of claims 1 to 4, wherein sodium hydroxide, potassium hydroxide, sodium carbonate or potassium carbonate or a mixture thereof is employed as the base.

6. The process as claimed in one or more of claims 1 to 5, wherein the reaction is carried out at a temperature of 60 to 160°C.

7. The process as claimed in one or more of claims 1 to 6, wherein the reaction is carried out at a temperature of 90 to 130°C.

8. The process as claimed in one or more of claims 1 to 7, wherein metallic copper, a copper(I) or copper(II) compound or a mixture thereof is employed as the copper.

9. The process as claimed in one or more of claims 1 to 8, wherein a copper(I) salt or a copper(II) salt or a mixture thereof is employed as the copper.

10. The process as claimed in one or more of claims 1 to 9, wherein 0.005 to 2.5% by weight of copper, based on the 2,4-dichlorobenzoic acid, is employed.

11. The process as claimed in one or more of claims 1 to 10, wherein 0.05 to 0.5% by weight of copper, based on the 2,4-dichlorobenzoic acid, is employed.

12. The process as claimed in one or more of claims 1 to 11, wherein pure oxygen or an oxygen-containing gas is employed as the oxygen.

13. The process as claimed in one or more of claims 1 to 12, wherein pure oxygen or an oxygen-containing gas is passed through the reaction mixture during the reaction.

14. The process as claimed in one or more of claims 1 to 13, wherein air is employed as the oxygen-containing gas.

15. The process as claimed in one or more of claims 1 to 14, wherein the reaction is carried out at a pH of 7.5 to 11.

16. The process as claimed in one or more of claims 1 to 15, wherein the reaction is carried out at a pH of 8 to 10.

17. The process as claimed in one or more of claims 1 to 16, wherein the pH is adjusted by addition of the base.

## Revendications

1. Procédé de préparation de la N-(2-carboxy-5-chloro-phényl)-glycine de formule (1) 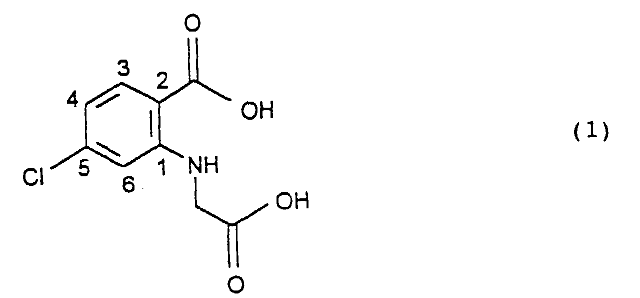 caractérisé en ce que qu'on fait réagir l'acide 2,4-dichlorobenzoïque de formule (2) 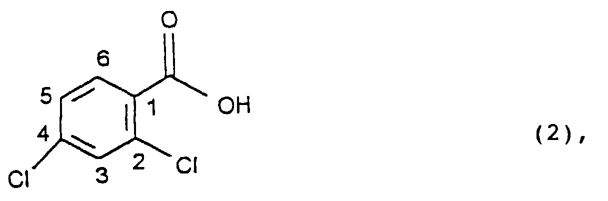 sur la glycine et une base dans l'eau, à une température de 50 à 200°C, en présence de cuivre et d'oxygène à un pH d'une valeur de 7 à 13.

2. Procédé selon la revendication 1, caractérisé en ce qu'on fait réagir l'acide 2,4-dichlorobenzoïque de formule (2) et la glycine dans un rapport molaire de 1:1 à 1:2.

3. Procédé selon la revendication 1 ou 2, caractérisé en ce qu'on utilise en tant que base un oxyde, un hydroxyde, un carbonate ou un bicarbonate d'un métal alcalin ou d'un métal alcalino-terreux ou un mélange de ces composés.

4. Procédé selon une ou plusieurs des revendications 1 à 3, caractérisé en ce qu'on utilise en tant que base un oxyde, un hydroxyde ou carbonate d'un métal alcalin ou un mélange de ces substances.

5. Procédé selon une ou plusieurs des revendications 1 à 4, caractérisé en ce qu'on utilise en tant que base l'hydroxyde de sodium, l'hydroxyde de potassium, le carbonate de sodium ou le carbonate de potassium ou un mélange de ces substances.

6. Procédé selon une ou plusieurs des revendications 1 à 5, caractérisé en ce qu'on met en oeuvre la réaction à une température de 60 à 160°C.

7. Procédé selon une ou plusieurs des revendications 1 à 6, caractérisé en ce qu'on met en oeuvre la réaction à une température de 90 à 130°C.

8. Procédé selon une ou plusieurs des revendications 1 à 7, caractérisé en ce qu'on utilise en tant que cuivre du cuivre métallique, un composé de cuivre(I) ou de cuivre(II)ou un mélange de ces substances.

9. Procédé selon une ou plusieurs des revendications 1 à 8, caractérisé en ce qu'on utilise en tant que cuivre un sel de cuivre(I) ou un sel de cuivre(II) ou un mélange de ces substances.

10. Procédé selon une ou plusieurs des revendications 1 à 9, caractérisé en ce qu'on utilise de 0,005 à 2,5 % en masse de cuivre, par rapport à l'acide 2,4-dichlorobenzoïque.

11. Procédé selon une ou plusieurs des revendications 1 à 10, caractérisé en ce qu'on utilise de 0,05 à 0,5 % en masse de cuivre, par rapport à l'acide 2,4-dichlorobenzoïque.

12. Procédé selon une ou plusieurs des revendications 1 à 11, caractérisé en ce qu'on utilise en tant qu'oxygène de l'oxygène pur ou un gaz contenant de l'oxygène.

13. Procédé selon une ou plusieurs des revendications 1 à 12, caractérisé en ce qu'on alimente le mélange réactionnelle au cours de la réaction en oxygène pur ou en un gaz contenant de l'oxygène.

14. Procédé selon une ou plusieurs des revendications 1 à 13, caractérisé en ce qu'on utilise l'air en tant que gaz contenant de l'oxygène.

15. Procédé selon une ou plusieurs des revendications 1 à 14, caractérisé en ce qu'on met en oeuvre la réaction à une valeur de pH de 7,5 à 11.

16. Procédé selon une ou plusieurs des revendications 1 à 15, caractérisé en ce qu'on met en oeuvre la réaction à une valeur de pH de 8 à 10.

17. Procédé selon une ou plusieurs des revendications 1 à 16, caractérisé en ce qu'on établit la valeur du pH par ajout de base.
